# EUROPEAN PATENT SPECIFICATION

(11) **EP 1 494 588 B1**
(45) Date of publication and mention of the grant of the patent: **07.06.2006**
(21) Application number: 03716530.5
(22) Date of filing: 12.03.2003
(51) Int. Cl.: A61B 10/00, G01N 33/94, B01L 3/00

(54) **DISK TESTING APPARATUS**
SCHEIBENFÖRMIGES PRÜFGERÄTS
DISPOSITIF DE TEST A DISQUE

(30) Priority: 08.04.2002 US 118832
(43) Date of publication of application: 12.01.2005
(73) Proprietor: Varian, Inc., Palo Alto, CA 94304 (US)
(72) Inventor: GALLOWAY, R., Keith, Fairfax Station, VA 22039 (US); BACHAND, Steven, S., Pocasset, MA 02559 (US)
(74) Representative: Kirschner, Klaus Dieter
(86) International application number: PCT/US2003/007719
(87) International publication number: WO 2003/086200

(56) References cited:
- WO-A-00/63697
- WO-A-01/89697
- US-A- 5 976 895

## Description

Present invention generally relates to diagnostic testing devices and more particularly is directed to a cup for containing a fluid sample, for example a urine specimen, and an insertion disk for providing indication of characteristics of the fluid specimen.

Fluid specimens, particularly body fluid such as urine, are usually collected and stored in cups or the like. Typically, cup is sealed with a lid which is thereafter punctured or removed in order to Transfer samples to a separate test apparatus.

During this procedure fluids can escape and cause contamination these user processing errors may also contribute to incorrect results.

Many devices have been developed and are commercially available for the storage and testing fluid samples however such devices do not provide simplicity and convenience for their use.

As an example, U.S. Patent No. 5,976,895 describes a drug abuse test kit which has a transparent cup-like Container for retaining a fluid sample to be tested and the open top end of the Container is closed by an inner closure insert seated within the open end. There is a slit in the inner closure insert to receive a multiple drug test card having a plurality of immunoassay test Strips thereon with visual endpoints to indicate presence or absence of a particular drug. The Container is provided with an outer cover to close and seal the Container when a sample therein is to be transported. This is a urine-testing cup that has a slotted cap in which the user must perform multiple manipulations for use. Sealing of the cup after testing requires even more manipulation.

U.S. Patent No. 5,916,815 describes another type of cup that requires vigorous shaking to run the test and U.S. 5,403,551 requires positioning the cap in the defined location, tipping the cup to a specific angle for a set period of time and then waiting until a test valid signal appears in order to Interpret the test.

None of these or other heretofore developed test cup devices represent a true one-step process. In the present invention, disk testing apparatus is introduced into previously collected fluid specimens within a cup and the result is read without further manipulation.

A drug testing collection cup in accordance with the present invention generally includes a cup body having a top, bottom and a flat sidewall with the flat sidewall being transparent, (such cups being known in the art, see WO 01/89697 for instance.) At least one test strip is provided for assaying a fluid specimen, such as, for example, urine, and visually displaying an assay result.

A disk is provided and sized for insertion into the cup for suspending the test strip within the cup proximate the flat sidewall at a distance enabling a visual perception of the assay result to the transparent flat sidewall.

A cap is provided for sealing the cup top to enable safe storage and transport thereof. Because of the proximity of the test strip to the transparent sidewalls, easy determination of the test results are determined without opening the cup. In addition, no agitation, manipulation or any other handling is required in order to effect an assay of the fluid specimen within the cup. More particularly, the flat sidewall extends from the cup bottom to a point proximate the cup top and the strip is disposed proximately parallel to the flat sidewall. This structural configuration enables a plurality of relatively long test strips to be utilized and observed through the flat of viewing surface of the sidewall.

Still more particularly, the disk includes at least one member, depending from a bottom of the disk, for releasably grabbing a test strip. A plurality of strips may be supported by the disk and may be, if desired, permanently attached to the disk. Preferably, however, the member for grasping the test strip enables test strips of different configuration to be utilized with the present invention with the final selection of particular assay strips being made by the user.

To facilitate the handling of the disk a finger grip is disposed on the top of the disk for manual insertion and removal of the disk, if desired, from the cup.

Preferably, the cup top is circular and includes threads therein for releasably engaging the cap. This configuration enables a fluid seal to be established. In addition, the disk may include a circular perimeter for facilitating its introduction to the cup by a user.

The cap may include means for engaging a disk upon sealing of the cap to the cup which enables removal of the disk with the cap as the cap is removed from the cup. This embodiment is preferred when storage or shipping of the cup is to be done without the test strips therein. In this case, a second cap is provided for sealing the cup after removal of the first cap and the disk from the cup. Alternatively, the test strip may be easily removed (pulled out) from the disk, leaving the disk in the cap. The cap/disk assembly can then be placed on the cup and tumed to seal the cup for shipping. The disk has a thin sealing surface on its perimeter that acts as a gasket between the cap and cup.

The advantages and features of the present invention will be better understood by the following description when considered in conjunction with the accompanying drawings in which::
Figure 1 is a perspective view of the drug testing collection cup in accordance with the present invention generally showing a cup having a flat side along with a disk positioned thereon;
Figure 2 is a view similar to Figure 1 illustrating a transparent cup and the positioning of a drug strip within the cup by the disk;
Figure 3 is a side view of the apparatus of Figures 1 and 2 shown in cross section along with a cap for sealing the cup;
Figure 4 is a side view of the disk in accordance with the present invention showing a means for removably grasping the test strips or cards and a finger grip for facilitating handling of the disk;
Figure 5 is a perspective top view of the disk showing in Figure 4;
Figure 6 is a perspective bottom view of the disk showing in Figure 4; and
Figure 7 is a bottom perspective view of the disk engaged with the cap to facilitate its removal from the cup.

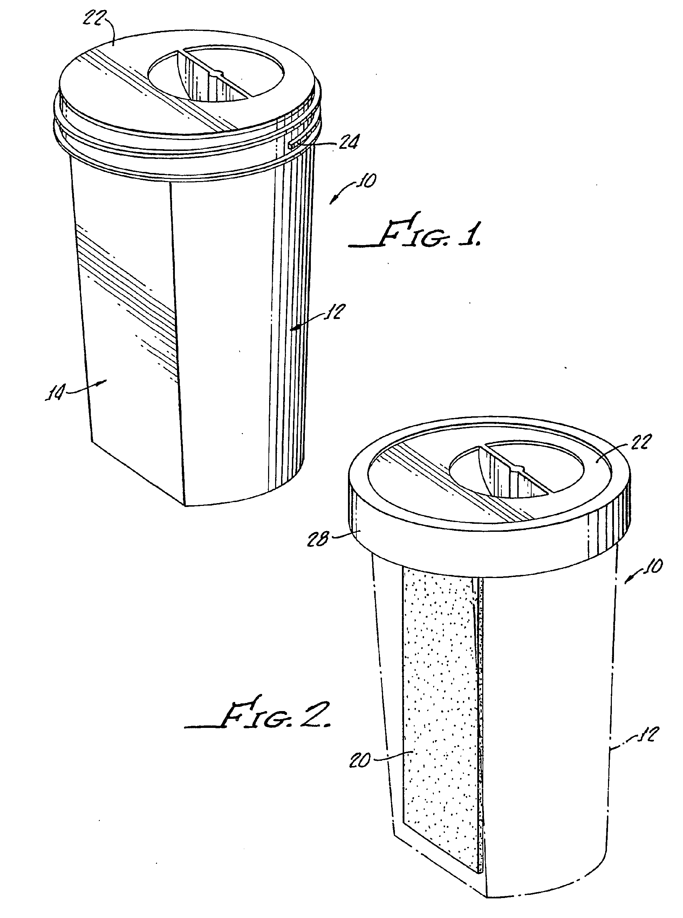

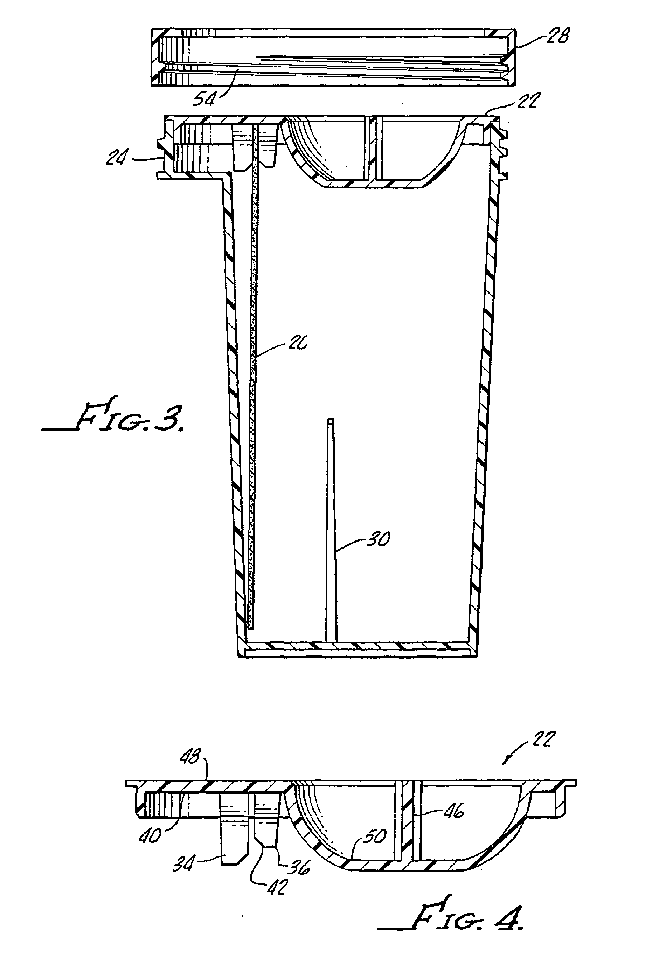

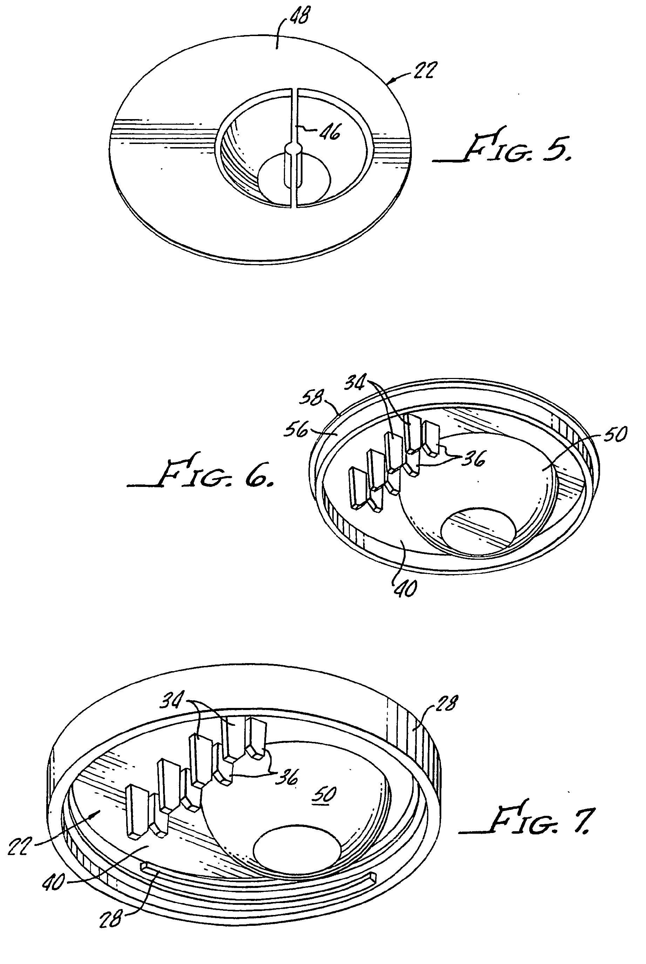

With reference to Figures 1-3 there is shown a drug testing collection cup 10 in accordance with the present invention which generally includes a cup body 12 which includes a flat sidewall 14 which is transparent. This provides a clear flat viewing configuration of a test strip or card 20 as shown in Figures 2 and 3.

A disk 22, see also Figure 4, is provided and size for insertion into a cup top 24, which suspends the strip 20 within the cup body 12 proximate the flat clear sidewall 14 at a distance enabling visual perception of the assay result exhibited by the test strip through the sidewall 14.

The cup 12 disk 22 and cap 28, see Figure 3, may be formed from any suitable material such as, for example but not limited to, polystyrene, polyethylene or polypropylene. The strip 20 may be of any suitable manufacturer for assaying a fluid sample, such as urine, and visually displaying an assay result.

Ribs 30 may be provided for preventing complete nesting of the cups 10 when stacked, one inside another, in order that locking of cups 10 to one another does not occur. Such stacking, enabled by the cup 10 configuration is preferable for storage and shipping considerations.

Rigidity of the sidewall 14 enables a spacing between the card or strip 20 and the sidewall of between about 2.5 mm (0.10 inches) and about 5 mm (0.20 inches). It should be appreciated that these dimensions are referenced to reflect actual design and it should be understood that other spacing distances may also be appropriate.

With particular reference to Figure 4 the disk 22 includes members 34, 36 which depend from an underside 40 of the disk and are preferably molded therein at a spaced apart distance for providing a gap 42 therebetween for receiving the strip, or card, 20, resiliency of the members 34, 36 enabling a pressure fit against the strip 20 enabling removal and adjustment of the strip 20 or strips in a parallel array. Also as shown in Figures 4-7 the disk 22 includes a finger grip 46 molded into a top 48 of the disk.

A depending well 50 provides for clearance for finger engagement with the grip 46. This arrangement enables handling of the disk while at the same time no portion protrudes above the disk top 48.

The cup body 12 has a circular top 24 which enables the cap 28 with threads 54 to be screwed thereon in order to seal the cup top 24. The disk 22 also preferably includes a circular perimeter and a ridge 58 that serves as a perimeter flange for sealing the surface between the cup, extending therefrom which provides a means for engaging the cap 28 upon screwing of the cap 28 onto the cup top 24. The engagement is caused by the forcing of the ridge 28 past the threads in the cap as shown in Figure 7.

Thus the strip 20 may be removed from the cup 12 by unscrewing of the cap 28 with the disk with the 28 attached thereto. Flexibility of the disk 22 enables the rotation thereof within the cap 28 and cup body 12 during strip 20 removal.

The cup 12 is thereafter sealed with a second cap, which may be identical to that shown in the Figures, and indicated with the character reference 28. Alternatively, after removing the strip 20 the disk 22 may be left in the cap 28 with the thin perimeter ridge or flange 58 acting as a seal.

While a ridge 58 and thread 54 provide a means for engaging the disk 22 and cap 28 it should be appreciated that any other suitable means for enabling engagement of the disk 22 and cap 28 are to be considered within the scope of the present invention.

However, the ridge 28 and cap threads 54 provide a simple economical means for engagement which may not be provided by other engagement devices, not shown.

Although there has been hereinabove described a drug testing collection in accordance with the present invention for the purpose of illustrating the manner in which the invention may be used to advantage, it should be appreciated that the invention is not limited thereto.

## Claims

1. A drug testing collection cup (12) in particular for urine collection comprising:
a cup body having a top, bottom and a flat sidewall (14), said flat sidewall (14) being transparent;
at least one test strip (20) for assaying a sample and visually displaying an assay result;
a disk (22), sized for insertion into the cup top, for suspending the test strip (20) within the cup body proximate the flat sidewall (14) at a distance enabling visual perception of the assay result through the transparent flat sidewall (14); and
a cap (28) for sealing the cup top for enabling transport thereof, wherein
the disk (22) has a thin sealing surface on its perimeter that acts as a gasket between the cap (28) and the cup (12).

2. The collection cup (12) according to claim 1 wherein the flat sidewall (14) extends from the cup bottom to a point proximate the cup top and the strip (20) is disposed approximately parallel to die flat sidewall (14).

3. The collection cup (12) according to claim 2 wherein the cup top is circular and includes thread thereon for releasably engaging said cap (28).

4. The collection cup (12) according to claim 3 wherein the disk (22) includes a circular perimeter for providing a seal between said cup (12) and said disk (22).

5. The collection cup (12) according to claim 1 comprising:
a first cap (28) for sealing the cup (12) top and engaging said disk (22), the engagement enabling removal of said disk (22), and test strip (20), from said cup (12); and
a second cap for sealing the cup top for enabling transport thereof.

6. The collection cup (12) according to claim 5 wherein the flat sidewall (14) extends from die cup bottom to a point proximate the cup (12) top and the strip (20) is disposed approximate parallel to die flat sidewall (14).

7. The collection cup (12) according to claim 6 wherein the cup top is circular and includes threads thereon for releasably engaging the first and second cap.

8. The collection cup (12) according to claim 3 or 7 wherein said disk (22) includes at least one member, depending from a bottom of said disk (22) for releasing grasping the test strip (20).

9. The collection cup (12) according to claim 8 wherein the disk (22) includes a perimeter flange for engaging threads in said cup (12) for providing engagement therebetween.

10. The collection cup (12) according to claim 5 wherein said disk (22) includes a finger grip, disposed on a top of said disk (22) for enabling manual insertion of said disk (22) into said cup (12).

11. The collection cup (12) according to claim 6 wherein at least two members, depending from the disk bottom, are adjacently disposed for enabling press-fit grasping of the test strip (20),

12. The collection cup (12) according to claim 11 further comprising means for engaging said disk (22) and cap (28) upon sealing of the cap (28) to said cup (12), for enabling removal of said disk (22) with said cap (28) as said cap (28) is removed from said cup (12).

13. The collection cup (12) according to claim 12 wherein the means for engaging the disk (22) and cap (28) includes a flange formed in the disk (22) and threads formed in the cap (28).

14. The collection cup (12) according to claim 13 wherein the disk (22) includes a circular perimeter for sealing said cap (28) to said cup (12).

## Patentansprüche

1. Arzneimitteltest-Sammelbehälter (12), insbesondere zum Sammeln von Urin, umfassend:
einen Behälterkörper mit einer Oberseite, einer Unterseite und einer flachen Seitenwand (14), wobei die flache Seitenwand (14) transparent ist;
wenigstens einen Teststreifen (20) zur Probennahme einer Probe und zur visuellen Darstellung eines Testresultats;
eine Scheibe (22), die zum Einfügen in die Behälteroberseite dimensioniert ist, um den Teststreifen (20) in dem Behälterkörper nahe bei der Seitenwand (14) mit einem Abstand aufzuhängen, der eine visuelle Erfassung des Testresultats durch die transparente flache Seitenwand (14) ermöglicht; und
eine Kappe (28) zum Abdichten der Behälteroberseite, um den Transport desselben zu ermöglichen, wobei
die Scheibe (22) eine dünne Dichtungsoberfläche an ihrem Umfang hat, die als Dichtung zwischen der Kappe (28) und dem Behälter (12) wirkt.

2. Sammelbehälter (12) gemäß Anspruch 1, worin die flache Seitenwand (14) sich von dem Behälterboden bis zu einem Punkt nahe bei der Behälteroberseite erstreckt, und worin der Streifen (20) nahezu parallel zu der flachen Seitenwand (14) angeordnet ist.

3. Sammelbehälter (12) entspricht Anspruch 2, worin die Behälteroberfläche kreisförmig ist und darauf ein Gewinde enthält, um die Kappe (28) lösbar in Eingriff zu bringen.

4. Sammelbehälter (12) entsprechend Anspruch 3, worin die Scheibe (22) einen kreisförmigen Umfang umfasst, um eine Dichtung zwischen dem Behälter (12) und der Scheibe (22) zu liefern.

5. Sammelbehälter (12) entsprechend Anspruch 1, umfassend
eine erste Kappe (28) zur Abdichtung der Oberseite des Behälters (12) und zum Angreifen an der Scheibe (22), wobei der Eingriff das Entfernen der Scheibe (22) und des Teststreifens (20) von dem Behälter (12) ermöglicht; und
eine zweite Kappe zur Abdichtung der Behälteroberseite, um den Transport derselben zu ermöglichen.

6. Sammelbehälter (12) entsprechend Anspruch 5, worin die flache Seitenwand (14) sich von dem Behälterboden bis zu einem Punkt nahe bei der Oberseite des Behälters (12) erstreckt, und worin der Streifen (20) nahezu parallel zu der flachen Seitenwand (14) angeordnet ist.

7. Sammelbehälter (12) entsprechend Anspruch 6, worin die Behälteroberseite kreisförmig ist und darauf ein Gewinde umfasst, um die erste und die zweite Kappe lösbar in Eingriff zu bringen.

8. Sammelbehälter (12) entsprechend Anspruch 3 oder 7, worin die Scheibe (22) wenigstens einen Teil aufweist, der von einem Boden der Scheibe (22) herunterhängt, um das Erfassen des Teststreifens (20) freizugeben.

9. Sammelbehälter (12) entsprechend Anspruch 8, worin die Scheibe (22) einen Umfangsflansch aufweist, um an einem Gewinde in dem Behälter (12) anzugreifen, um einen Eingriff dazwischen bereitzustellen.

10. Sammelbehälter (12) entsprechend Anspruch 5, worin die Scheibe (22) einen Fingergriff aufweist, der auf einer Oberseite der Scheibe (22) angeordnet ist, um das Einführen der Scheibe (22) von Hand in den Behälter (12) zu ermöglichen.

11. Sammelbehälter (12) entsprechend Anspruch 6, worin wenigstens zwei Teile, die von der Unterseite der Scheibe herunterhängen, nebeneinander angeordnet sind, um eine Erfassung des Teststreifens (20) mit einem Presssitz zu ermöglichen.

12. Sammelbehälter (12) entsprechend Anspruch 11, ferner umfassend Mittel zum Angreifen an der Scheibe (22) und der Kappe (28) beim Abdichten der Kappe (28) an dem Behälter (12), um das Entfernen der Scheibe (22) mit der Kappe (28) zu gestatten, wenn die Kappe (28) von dem Behälter (12) entfernt wird.

13. Sammelbehälter (12) entsprechend Anspruch 12, worin die Mittel zum Angreifen an der Scheibe (22) und der Kappe (28) einen Flansch, der in der Scheibe (22) ausgebildet ist, und ein Gewinde umfassen, das in der Kappe (28) ausgebildet ist.

14. Sammelbehälter (12) entsprechend Anspruch 13, worin die Scheibe (22) einen kreisförmigen Umfang zum Abdichten der Kappe (28) an dem Behälter (12) umfasst.

## Revendications

1. Tasse de collection pour test de drogues (12), particulièrement pour collection d'urine comprenant :
- un corps de tasse ayant une partie supérieure, une base et une paroi latérale plate (14), ladite paroi latérale plate (14) étant transparente;
- au moins une bande de test (20) pour tester un échantillon et montrer visuellement un résultat du test;
- un disque (22), dimensionné pour l'insertion dans la partie supérieure de la tasse, pour suspendre la bande de test (20) dans le corps de la tasse proche de la paroi latérale (14) à une distance permettant la perception visuelle du résultat du test par ladite paroi latérale plate transparente (14) ; et
- un couvercle (28) pour étancher la partie supérieure de la tasse pour permettre le transport de celle-ci, où
- le disque (22) présente une surface d'étanchéité mince sur son périmètre qui actionne comme une garniture d'étanchéité entre le couvercle (28) et la tasse (12).

2. Tasse de collection (12) selon la revendication 1 où la paroi latérale plate (14) s'étend à partir de la base de la tasse à un point proche de la partie supérieure de la tasse et la bande (20) est disposée approximativement parallèle à la paroi latérale plate (14).

3. Tasse de collection (12) selon la revendication 2 où la partie supérieure de la tasse est circulaire et comprend un filet sur celle-ci pour engager de manière démontable ledit couvercle (28).

4. Tasse de collection (12) selon la revendication 3 où le disque (22) comprend un périmètre circulaire pour pourvoir un étanchement entre ladite tasse (12) et ledit disque (22).

5. Tasse de collection (12) selon la revendication 1 comprenant:
- un premier couvercle (28) pour étancher la partie supérieure de la tasse (12) et pour engager ledit disque (22), l'engagement permettant l'éloignement dudit disque (22), et de la bande de test (20), de ladite tasse (12); et
- un second couvercle pour étancher la partie supérieure de la tasse pour permettre le transport de celle-ci.

6. Tasse de collection (12) selon la revendication 5, où la paroi latérale plate (14) s'étend à partir de la base de la tasse à un point proche de la partie supérieure de la tasse (12) et la bande (20) est disposée approximativement parallèle à la paroi latérale plate (14).

7. Tasse de collection (12) selon la revendication 6, où la partie supérieure de la tasse est circulaire et comprend des filets sur celle-ci pour engager de manière démontable le premier et le second couvercle.

8. Tasse de collection (12) selon la revendication 3 ou 7, où ledit disque (22) comprend au moins une pièce, se reposant sur une base dudit disque (22) pour délivrer de fixation la bande de test (20).

9. Tasse de collection (12) selon la revendication 8, où le disque (22) comprend un rebord sur le périmètre pour engager des filets dans ladite tasse (12) pour pourvoir un engagement là entre.

10. Tasse de collection (12) selon la revendication 5, où ledit disque (22) comprend un élément de fixation, disposé sur une partie supérieure dudit disque (22) pour permettre l'insertion manuelle dudit disque (22) dans ladite tasse (12).

11. Tasse de collection (12) selon la revendication 6, où au moins deux pièces, se reposant à partir de la base du disque, sont disposées en voisinage pour permettre la fixation correspondante par pression de la bande de test (20).

12. Tasse de collection (12) selon la revendication 11, comprenant de plus des moyens pour engager ledit disque (22) et couvercle (28) à l'étanchement du couvercle (28) à ladite tasse (12), pour permettre l'éloignement dudit disque (22) avec ledit couvercle (28) quand ledit couvercle (28) est éloigné de ladite tasse (12).

13. Tasse de collection (12) selon la revendication 12, où le moyen pour engager le disque (22) et le couvercle (28) comprend un rebord formé dans le disque (22) et des filets formés dans le couvercle (28).

14. Tasse de collection (12) selon la revendication 13, où le disque (22) comprend un périmètre circulaire pour étancher ledit couvercle (28) à ladite tasse (12).
